# EUROPEAN PATENT SPECIFICATION

(11) **EP 2 464 968 B1**
(45) Date of publication and mention of the grant of the patent: **15.04.2015**
(21) Application number: 10745196.5
(22) Date of filing: 10.08.2010
(51) Int. Cl.: G01N 33/50

(54) **INSECT-BASED MODEL FOR PHARMACO-KINETIC STUDIES**
INSEKTENBASIERTES MODELL FÜR PHARMAKOKINETISCHE STUDIEN
MODÈLES À BASE D INSECTES POUR DE ÉTUDES DE PHARMACOCINÉTIQUE

(30) Priority: 12.08.2009 US 233153 P
(43) Date of publication of application: 20.06.2012
(73) Proprietor: Entomopharm APS, 5250 Odense SV (DK)
(72) Inventor: AADAL NIELSEN, Peter, S-23840 Oxie (SE); ANDERSSON, Gunnar, S-26024 Roestaanga (SE); ANDERSSON, Olga, S-26024 Roestaanga (SE)
(74) Representative: Zacco Denmark A/S
(86) International application number: PCT/EP2010/061593
(87) International publication number: WO 2011/018450

(56) References cited:
- HE S X ETAL.,: "Benzo(a)pyrene toxicokinetics in the cricket following injection into the haemolymph" ENVIRONMENTAL TOXICOLOGY AND PHARMACOLOGY, vol. 6, October 1998 (1998-10), pages 81-89, XP002601386 DOI: 10.1016/S1382-6689(98)00021-0
- HAMAMOTO H ET AL: "Silkworm as a model animal to evaluate drug candidate toxicity and metabolism" COMPARATIVE BIOCHEMISTRY AND PHYSIOLOGY PART C: TOXICOLOGY & PHARMACOLOGY, ELSEVIER LNKD- DOI:10.1016/J.CBPC.2008.08.008, vol. 149, no. 3, 1 April 2009 (2009-04-01) , pages 334-339, XP025988606 ISSN: 1532-0456 [retrieved on 2008-09-06]
- HAMAMOTO H ET AL: "Quantitative evaluation of the therapeutic effects of antibiotics using silkworms infected with human pathogenic microorganisms" ANTIMICROBIAL AGENTS AND CHEMOTHERAPY, AMERICAN SOCIETY FOR MICROBIOLOGY, WASHINGTON, DC, US LNKD- DOI:10.1128/AAC.48.3.774-779.2004, vol. 48, no. 3, 1 March 2004 (2004-03-01), pages 774-779, XP002989197 ISSN: 0066-4804
- TARANI KANTA BARMAN ET AL: "Utilization of Bombyx mori larvae as a surrogate animal model for evaluation of the anti-infective potential of oxazolidinones" JOURNAL OF INFECTION AND CHEMOTHERAPY ; OFFICIAL JOURNAL OF THE JAPANESE SOCIETY OF CHEMOTHERAPY AND THE JAPANESE ASSOCIATION FORINFECTIOUS DISEASES, SPRINGER-VERLAG, TO, vol. 14, no. 2, 30 April 2008 (2008-04-30) , pages 166-169, XP019595394 ISSN: 1437-7780

## Description

### FIELD OF THE INVENTION

The present invention is directed to methods using an insect model that is aimed to reflect vertebrate pharmaco-kinetics (PK). This model is useful for efficient screening of and assessment of PK profiles of newly synthesized compounds in the early phase of drug discovery.

### BACKGROUND OF THE INVENTION

Pharmacokinetics is the study of the fate of pharmaceuticals and other biologically active compounds from the time they are introduced into the body until they are eliminated. For example, the sequence of events for an oral drug can include absorption through the various mucosal surfaces, distribution via the blood stream to various tissues, biotransformation in the liver and other tissues, action at the target site, and elimination of drug or metabolites in urine or bile. Pharmacokinetics provides a rational means of approaching the elimination of a compound in a biological system.

One of the fundamental challenges researchers face in drug, environmental, nutritional, consumer product safety, and toxicology studies is the extrapolation of ADME (absorption, distribution, metabolism, and excretion) data and risk assessment from in vitro cell culture assays to animals, including humans. Although some conclusions can be drawn with the application of appropriate pharmacokinetic principles, there are still substantial limitations. One concern is that current screening assays utilize cells under conditions that do not replicate their function in their natural setting. The circulatory flow, interaction with other tissues, and other parameters associated with a physiological response are not found in standard tissue culture formats. For example, in a macroscale cell culture analog (CCA) system, cells are grown at the bottom of chambers. These systems have non-physiological high liquid-to-cell ratios, and have an unrealistic ratio of cell types (e.g., ratio of liver to lung cells). In a variant form of the macroscale CCA system the cells are grown on microcarrier beads. These systems more closely resemble physiological conditions, but are still deficient because they do not mimic physiological conditions accurately enough for predictive studies. Therefore, the resulting assay data is not based on the pattern of drug or toxin exposure that would be found in an animal.

Within living beings, concentration, time and elimination interact to influence the intensity and duration of a pharmacologic or toxic response. For example, in vivo the presence of liver function strongly affects drug metabolism and bioavailability. Elimination of an active drug by the liver occurs by biotransformation and excretion. Biotransformation reactions include reactions catalyzed by the cytochrome P450 enzymes, which transform many chemically diverse drugs. A second biotransformation phase can add a hydrophilic group, such as glutathione, glucuronic acid or sulfate, to increase water solubility and speed up the elimination through the kidneys.

While biotransformation can be beneficial, it may also have undesirable consequences. Toxicity results from a complex interaction between a compound and the organism. During the process of biotransformation, the resulting metabolite can be more toxic than the parent compound and the single-cell assays used for toxicity screening miss these complex inter-cellular and inter-tissue effects.

Traditional methods of predicting human response utilize surrogates typically either static, homogeneous in vitro cell culture assays or in vivo animal studies. In vitro cell culture assays are of limited value because they do not accurately mimic the complex environment a drug candidate is subjected to within a human and thus cannot accurately predict metabolic fate of the drug or the human risk. Similarly, while in vivo animal testing can account for these complex inter-cellular and inter-tissue effects not observable from in vitro cell-based assays, in vivo animal studies are extremely expensive, labor-intensive, and time consuming.

He et al, 1998 discloses a method for assessing a pharmaco-kinetic profile of a chemical compound by administring a compound into the hemolymph of a locust, periodically taking haemolymph samples, determining the concentration of the chemical compound and plotting the PK profile. Hamamoto et al., 2009 also discloses such a method using silk-worm. However, neither in the method of He et al, 1998 nor those of Hamamoto et al., 2009 are the hemolymph samples from a single living insect.

It is therefore an object of the present invention to provide an attractive alternative to animal studies without compromising the validity of the studies.

### SUMMARY OF THE INVENTION

The present invention provides new methodology for initial assessment of compound PK. The invention is generally useful for efficient screening of and assessment of PK profiles of newly synthesized compounds in the early phase of drug discovery.

Specifically, there is provided a method for determining a pharmaco-kinetic profile of a chemical compound, such as a drug, in vertebrates, said method comprises the steps of:
- administering a chemical compound either intra intestinal, oral or by injection into the hemolymph of an insect;
- periodically taking hemolymph samples;
- determining the concentration of the chemical compound in the hemolymph samples; and
- plotting the PK profile of the drug, and
- optionally sacrificing the insect.

In a preferred embodiment of the present invention the hemolymph is enriched in vivo with albumin before treatment with the chemical compound, which preferably is a drug, to make the method relevant for studying PK of drugs in situations with low and high protein binding (free and protein bound drugs).

In a particularly preferred embodiment of the present invention the insect is selected from the group consisting of the orders Dictyoptera, Orthoptera, Cheleutoptera, Hymenoptera, Odonata, Lepidoptera and Diptera.

It is preferred that the chemical compound is administered to the insect and the fate of the agent is studied for 1 min. - 72 hrs In this respect the concentration of the chemical compound preferably is 0.1 ng/ml - 50 mg/ml. Preferably the chemical compound is administered as a solution.

Preferable the concentration of the chemical compound is determined by LC/MS. In this respect the determination of the concentration of the chemical compound is performed by homogenizing or ultra sound disintegration (UD) the dissected organ, centrifugation and analyzing the concentration of the chemical compound in the supernatant by liquid chromatography with mass spectrometric detection of the eluted compounds.

The present invention is directed to the determination of a PK profile of a chemical compound. As may be evident to a skilled person the compound may be a drug or other chemical compound.

Similar to vertebrates, cells in the insect organism require an optimal environment to fulfil their functions. This involves the maintenance of a constant level of salt and water and osmotic pressure in the hemolymph of the insect. Since insects in general have a large surface to volume ratio they live under continual osmotic stress and for terrestrial insects the defence against desiccation is a major challenge. The insect excretory system (composed of Malpighian tubules and hindgut) is a major player that dynamically balances primary urine generation and secondary reabsorption to achieve a compromise between osmoregulation and excretion.

Malpighian tubues are long thin (one cell thick) blindly ending tubes arsing from the gut near the junction of midgut and hindgut and laying freely in the hemolymph in the body cavity. Their numbers vary and in some species they are very abundant (e.g in the desert locust Schistocera about 250) indicating a most efficient capacity to exchange material with the hemolymph and maintain a proper internal environment by osmoregulation. The principal cells in the Malpighian tubule are laterally held together by septate junctions and towards the luminal site they are enriched with close-packed microvilli and the cells contain numerous mitochindria indicating metabolically very active cells.

In all insects, the movement of water into the Malpighian tubules from hemolymph depends on the active transport of cations (predominantly potassium) into the lumen of the tubule. Solutes in the hemolymph will move in by passive diffusion down the concentration gradient as well as by active pumping. Passive diffusion occurs both between the cells (paracellular) and through the cells (transcellular route). Transcellular movements are slow due to the septate junctions and the small area compared to total outer surface area. All solutes in the hemolymph tend to diffuse into the tubule paracellularly. Large molecules are unable to pass through the cell membrane unless actively transported and they can therefore only pass paracellularly. This is also true for positively charged molecules while small uncharged molecules are able to diffuse transcellularly. Thus the principles for molecular entry to the Malpighial lumen are the same as for the entry of molecules during primary urine formation in vertebrates.

Reabsoption occurs in both the Malpighian tubule and in the hindgut. Thus, e.g. glucose is reabsorbed from the Malpighian tubule in the Locusta migratoria while amino acids may be actively reabsorbed from the hindgut (e.g. proline in Schistocera).

All insects have some innate capacity to metabolize toxic compounds, convert lipophilic compounds into water soluble products and excrete them. Many different enzymes are known to be involved in these reactions but a major player is the cytochrome P-450. The broad specificity of the P-450 system makes it active against a range of toxic or xenobiotic compounds. These processes may occur in a variety of tissues as there is no organ comparable to the vertebrate liver in the insects. The activities of the appropriate enzymes occur mainly in the midgut, fat body and Malpighian tubule. In a recent microarray study in Drosophila (Wang et al., 2004) it was confirmed that the tubule is remarkable enriched in detoxification genes, notably P-450s and glutathione S-transferases. Despite a 400 million years of divergent evolution there are striking similarities between the insect and human renal system. Thus the epithelial V-ATPase system to energize fluid secretion in insects is also highly expressed in multiple vertebrate epithelial cells (Wieczorek et al., J.Exp. Biol., 2009; Wieczorek et al., BioEssays., 1999). Also the Na+, K+-ATPase-dominated picture in mammals is also found to play a major role in insect tubule function (lanowski and O'Donnell, J Exp. Biol., 2004; Torrie et al., PNAS, 2004). Once the xenobiotics have been metabolized or otherwise rendered soluble, the Malpighian tubule may transport them onwards. By analogy with vertebrates attention has been focused also in insects on the multiple drug resistant P-glycoprotein ABC transporter. It was clearly shown in the microarray study (Wang et al., 2004) that nearly every subclass of the huge ABC transporter gene family as well as the OAT, OATP, sugar, multivitamin and amino acid transporter families are very highly expressed in the Malpighian tubule. Thus, there are strong evidences to believe that the insect hemolymph- Malpighian metabolizing-excretion system is a relevant model for highly efficient studies of early compound PK documentation and selection.

### DETAILED DESCRIPTION OF THE INVENTION

The present invention provides new methodology for initial assessment of compound PK. The invention is generally particular useful for efficient screening of and assessment of PK profiles of newly synthesized compounds in the early phase of drug discovery.

A drug in accordance with the present invention is defined in its broadest scope as a chemical compound that, when absorbed into the body of a living organism, alters normal bodily function. More specifically, a drug in accordance with the present invention is a chemical compound that may be used in the treatment, cure, prevention, or diagnosis of disease or used to otherwise to enhance physical or mental well-being. Of particular interest in accordance with the present invention are psychoactive drugs, which are chemical compounds that cross the BBB and acts primarily upon the central nervous system where it alters brain function, resulting in changes in perception, mood, consciousness, cognition and behavior.

The present invention relates to but is not restricted to the use of insects selected from the following orders (Taxonomy according to: Djurens Värld, Ed B. Hanström; Förlagshuset Norden AB, Malmö, 1964).

| **Order** | **Suborder/family** | **Comment** |
|---|---|---|
| Dictyoptera | Blattoidea | Cockroach |
| | Mantoidea | |
| Orthoptera | Grylloidea | Crickets |
| | Acridoidea | Grasshoppers |
| Cheleutoptera | | Stick insects |
| Lepidoptera | | Moths |
| Hymenoptera | Formicoidea | Ants |
| | Vespoidea | Wasps |
| | Apoidea | Bee like hymenopterans |
| | Bombinae | Bumble-bees |
| | Apine | Proper bees |
| Odonata | | Dragonflies |
| Diptera | Nematocera | Mosquitos |
| | Brachycera | Flies E.g Drosophila |

In particular the invention relates to insect species selected from Blattoidea, Acridoidea, Cheleutoptera, Brachycera and Lepidoptera and most particular to the Acridoidea (Locusta migratoria and Schistocera gregaria).

The invention will also relate to the following orders comprising insect species relevant for the method of the present invention:

| **Order** | **Suborder/family** | **Comment** |
|---|---|---|
| Ephemerida | | Mayflies |
| Plecoptera | | |
| Dermoptera | Forficuloidea | Earwigs |
| Homoptera | Cicadinea | Cicadas |
| | Aphidine | Plant-louse |
| Heteroptera | | Hemipteran |
| Coleoptera | | Beetles |
| Trichoptera | | Caddis fly |

The present invention preferably uses large insects, such as the migratory locust, Locusta migratoria and the desert locust, Schistocera gregaria or cockroach where it's feasible to administer test compounds and subsequently take hemolymph for analyses. The locust has been used to develop the model to determine the PK of different therapeutic drugs and compare the PK profiles with existing literature data from conventional in vivo vertebrate studies.

In the present invention specific insects are used as model systems in order to improve compound selection processes and reduce the costs during the drug discovery phase. Based on experiments it has been found that the insect model provide a better foundation for a high throughput PK screening and cost efficient assessment and selection of compounds in the early discovery phase than existing in vivo models.

Accordingly, the present invention focuses on insect models that are aimed to reflect the vertebrate PK profile of the test compounds. Investigation of the PK profile is of extreme importance in compound selection during the early phase of drug discovery.

In accordance with a preferred embodiment of present invention the migratory locust, Locusta migratoria and/or the desert locust, Schistocera gregaria, is used since it is easy to breed and it is a relatively large insect with relevant hemolymph volumes and well documented and efficient Malpighian functions.

The application of a test substance to insects of the present invention in a PK screening method may be as follows, in accordance with a preferred embodiment of present invention.

### EXAMPLES

In a preferred embodiment of present invention the insects are selected from the order Acridoidea and specifically Locusta migratoria and Schistocera gregaria are used. The insects may be obtained from local suppliers or bred in house. The grasshoppers were reared under crowded conditions at 28°C and a 12:12 dark:light photocycle and fed fresh grass and bran. Before experiments the grasshoppers were fed ecologically cultivated wheat for two weeks. Animals used are adult males (in some experiments females) between two to four weeks after adult emergence. Test compounds are administrated into the hemolymph by use of a Hamilton syringe and inserting the needle between two abdominal terga or administered intraintestinal by use of a catheter or a steel probe. At various time points after administration hemolymph samples are taken for quantitative determination of drug concentration in the hemolymph. The samples are snap-frozen and stored until analyses. Drug concentration is analysed by HPLC, LC/MSMS or other methods. In studies of drug retention various organs e.g. heart, fat bodies and brain are dissected at various times after drug administration. The tissues are washed, snap-frozen and stored until analyses. At analyses the tissues are homogenized/vortexed/sonicated and centrifuged. The drug concentration in the tissues is determined as above.

In the following the present invention is exemplified in further detail.

### Example 1

40 ul of an approximately 5 mg/ml solution of quinidine was injected into the hemolymph of Locusts (Locusta migratoria). The test compound was administered by using a Hamilton syringe. At 5, 15, 45, 120, and 360 minutes after administration (10 ul) hemolymph was collected from each locust with a calibrated capillary tube by puncturing the ventral membrane between the head and the thorax and immediately blown into a tube containing 40 ul aqua dest and 100 ul of acetonitrile. Each sample was centrifuged for 5 minutes (10 000g at 4°C) and 100 ul of the supernatants were transferred to new test tubes for LCMS analysis. The measured average hemolymph concentrations of quinidine are listed in table 1:

**Table 1**

| **Time (minutes)** | **Average hemolymph concentration of quinidine (in ng/ml)** |
|---|---|
| **5** | 3472 |
| **15** | 1715 |
| **45** | 1277 |
| **120** | 797 |
| **360** | 482 |

### Example 2

40 ul of an approximately 5 mg/ml solution of propranolol was injected into the hemolymph of Locusts (Locusta migratoria). The test compound was administered by using a Hamilton syringe. At 5, 15, 45, 120, and 360 minutes after administration (10 ul) hemolymph was collected from each locust with a calibrated capillary tube by puncturing the ventral membrane between the head and the thorax and immediately blown into a tube containing 40 ul aqua dest and 100 ul of acetonitrile. Each sample was centrifuged for 5 minutes (10 000g at 4°C) and 100 ul of the supernatants were transferred to new test tubes for LCMS analysis. The measured average hemolymph concentrations of propranolol are listed in table 2:

**Table 2**

| **Time (minutes)** | **Average hemolymph concentration of propranolol (in ng/ml)** |
|---|---|
| **5** | 821 |
| **15** | 982 |
| **45** | 469 |
| **120** | 190 |
| **360** | 48 |

### Example 3

40 ul of an approximately 5 mg/ml solution of caffeine was injected into the hemolymph of Locusts (Locusta migratoria). The test compound was administered by using a Hamilton syringe. At 5, 15, 45, 120, and 360 minutes after administration (10 ul) hemolymph was collected from each locust with a calibrated capillary tube by puncturing the ventral membrane between the head and the thorax and immediately blown into a tube containing 40 ul aqua dest and 100 ul of acetonitrile. Each sample was centrifuged for 5 minutes (10 000g at 4°C) and 100 ul of the supernatants were transferred to new test tubes for LCMS analysis. The measured average hemolymph concentrations of caffeine are listed in table 3:

**Table 3**

| **Time (minutes)** | **Average hemolymph concentration of caffeine (in ng/ml)** |
|---|---|
| **5** | 9285 |
| **15** | 9012 |
| **45** | 8775 |
| **120** | 6825 |
| **360** | 5498 |

### Example 4

40 ul of an approximately 5 mg/ml solution of atenolol was injected into the hemolymph of Locusts (Locusta migratoria). The test compound was administered by using a Hamilton syringe. At 5, 15, 45, 120, and 360 minutes after administration (10 ul) hemolymph was collected from each locust with a calibrated capillary tube by puncturing the ventral membrane between the head and the thorax and immediately blown into a tube containing 40 ul aqua dest and 100 ul of acetonitrile. Each sample was centrifuged for 5 minutes (10 000g at 4°C) and 100 ul of the supernatants were transferred to new test tubes for LCMS analysis. The measured average hemolymph concentrations of atenolol are listed in table 4:

**Table 4**

| **Time (minutes)** | **Average hemolymph concentration of atenolol (in ng/ml)** |
|---|---|
| **5** | 21598 |
| **15** | 19980 |
| **45** | 10258 |
| **120** | 3535 |
| **360** | 280 |

The test compounds in example 1- 4 were selected due to their different vertebrate ADME characteristics. Table 1- 4 summarizes the change in hemolymph concentration of the test compounds in the period 5 minutes to 6 hours after administration.

Compared to quinidine, caffeine and atenolol there is a marked reduction in propranolol hemolymph concentration. The difference between the two beta blockers propranolol and atenolol is expected since it has been shown in human that propranolol has a much larger volume of distribution and much shorter half life (indicating extensive metabolism) compared to atenolol. Both quinidine and caffeine are class 1 compounds but with much lower volume of distribution compared to propranolol. Furthermore, in a study by Liu et al., 2005, it was shown in a mouse study that the plasma clearance of propranolol was more that ten times faster compared to caffeine.

Conclusion: The hemolymph clearance of the test compound in the locust model strongly correlate to corresponding observations in human and vertebrate experimental models. The test compounds are characterized by differences in classification according to the Biopharmaceutics Classification System (quinidine, propranolol and caffeine being class 1 compounds differing in volume of distribution and metabolism and atenolol being a class 3 compound with small distribution volume and low permeability). All these vertebrate criteria are found in the locust model justifying this model as a test model in drug kinetics.

### Example 5

Mianserin was injected into the hemolymph of Locusts (Locusta migratoria). The administered volume was 40 ul (administered by using a Hamilton syringe) at a concentration of 8.8 mg/ml. At 5, 15 and 45 minutes after administration (2x20 ul) hemolymph was collected from the locusts with a calibrated capillary tube by puncturing the ventral membrane between the head and the thorax and immediately blown into a tube containing 60 ul aqua dest and 200 ul of acetonitrile. Each sample was centrifuged for 5 minutes (10 000xg at 4oC) and 100 ul of the supernatants were transferred to new test tubes for LCMS analysis. The average hemolymph concentrations at 5, 15 and 45 minutes were 37900, 17400 and 13020 ng/ml.

**Table 5**

| **Time (minutes)** | **Average hemolymph concentration of mianserin (in ng**/**ml**) |
|---|---|
| **5** | 37900 |
| **15** | 17400 |
| **45** | 13020 |

### Example 6

40 ul of an approximately 10 mg/ml solution of caffeine was injected into the hemolymph of Locusts (Locusta migratoria). The test compound was administered by using a Hamilton syringe. At 5, 15, and 45 minutes after administration (10 ul) hemolymph was collected from each locust with a calibrated capillary tube by puncturing the ventral membrane between the head and the thorax and immediately blown into a tube containing 40 ul aqua dest and 100 ul of acetonitrile. Each sample was centrifuged for 5 minutes (10 000g at 4°C) and 100 ul of the supernatants were transferred to new test tubes for LCMS analysis. The measured average hemolymph concentrations of caffeine are listed in table 6:

**Table 6**

| **Time (minutes)** | **Average hemolymph concentration of caffeine (in ng/ml)** |
|---|---|
| **5** | 29000 |
| **15** | 24000 |
| **45** | 23000 |

### Example 7

40 ul of an approximately 10 mg/ml solution of trazodone was injected into the hemolymph of Locusts (Locusta migratoria). The test compound was administered by using a Hamilton syringe. At 5, 15, and 45 minutes after administration (10 ul) hemolymph was collected from each locust with a calibrated capillary tube by puncturing the ventral membrane between the head and the thorax and immediately blown into a tube containing 40 ul aqua dest and 100 ul of acetonitrile. Each sample was centrifuged for 5 minutes (10 000g at 4°C) and 100 ul of the supernatants were transferred to new test tubes for LCMS analysis. The measured average hemolymph concentrations of trazodone are listed in table 7:

**Table 7**

| **Time (minutes)** | **Average hemolymph concentration of trazodone (in ng**/**ml**) |
|---|---|
| **5** | 13800 |
| **15** | 13600 |
| **45** | 5000 |

### Example 8

40 ul of an approximately 10 mg/ml solution of busprione was injected into the hemolymph of Locusts (Locusta migratoria). The test compound was administered by using a Hamilton syringe. At 5, 15, and 45 minutes after administration (10 ul) hemolymph was collected from each locust with a calibrated capillary tube by puncturing the ventral membrane between the head and the thorax and immediately blown into a tube containing 40 ul aqua dest and 100 ul of acetonitrile. Each sample was centrifuged for 5 minutes (10 000g at 4°C) and 100 ul of the supernatants were transferred to new test tubes for LCMS analysis. The measured average hemolymph concentrations of buspirone are listed in table 8:

**Table 8**

| **Time (minutes)** | **Average hemolymph concentration of buspirone (in ng/ml)** |
|---|---|
| **5** | 10700 |
| **15** | 6500 |
| **45** | 3800 |

### Example 9

40 ul of an approximately 10 mg/ml solution of haloperidol was injected into the hemolymph of Locusts (Locusta migratoria). The test compound was administered by using a Hamilton syringe. At 5, 15, and 45 minutes after administration (10 ul) hemolymph was collected from each locust with a calibrated capillary tube by puncturing the ventral membrane between the head and the thorax and immediately blown into a tube containing 40 ul aqua dest and 100 ul of acetonitrile. Each sample was centrifuged for 5 minutes (10 000g at 4°C) and 100 ul of the supernatants were transferred to new test tubes for LCMS analysis. The measured average hemolymph concentrations of haloperidol are listed in table 9:

**Table 9**

| **Time (minutes)** | **Average hemolymph concentration of haloperidol (in ng/ml)** |
|---|---|
| **5** | 2500 |
| **15** | 1500 |
| **45** | 800 |

### Example 10

40 ul of an approximately 10 mg/ml solution of loperamide was injected into the hemolymph of Locusts (Locusta migratoria). The test compound was administered by using a Hamilton syringe. At 5, 15, and 45 minutes after administration (10 ul) hemolymph was collected from each locust with a calibrated capillary tube by puncturing the ventral membrane between the head and the thorax and immediately blown into a tube containing 40 ul aqua dest and 100 ul of acetonitrile. Each sample was centrifuged for 5 minutes (10 000g at 4°C) and 100 ul of the supernatants were transferred to new test tubes for LCMS analysis. The measured average hemolymph concentrations of loperamide are listed in table 10:

**Table 10**

| **Time (minutes)** | **Average hemolymph concentration of loperamide (in ng/ml)** |
|---|---|
| **5** | 5600 |
| **15** | 3000 |
| **45** | 1700 |

The results for caffeine in example 6 shows the same change in hemolymph concentration as in example 3 confirming the reproducibility of the model and the non dose dependent hemolymph clearance (double dose compared to example 1). Haloperidol is a Class 2 according to the classification by Drug Disposition Classification System which implies low solubility but extensive metabolism. Compared to caffeine haloperidol is faster eliminated from the hemolymph and this is also the case for loperamide another less soluble compound. Trazodon is stable for 15 minutes and buspirone as a Class 1 compound also shows increased elimination similar to haloperidol.

The results in example 1 to 10 show that different compounds with different pharmacokinetic properties show different locust hemolymph kinetic patterns after intahemolymphic administration. The different patterns correlate to the classification of the characteristics of the compound and thus to the patterns in vertebrate models. Therefore these examples further strengthen the locust model as a relevant model for early characterization of test compound kinertics.

### REFERENCES

Wang J et al. (2004) Function-informed transcriptome analysis of Drosophila renal tubule. Genome Biol 5: R69.
Wieczorek H et al. (1999) Animal plasma membrane energization by proton motive V-ATPases. BioEssays 21: 637-648.
Torrie L S et al. (2004) Resolution of the insect oubain paradox. PNAS 101: 13689-13693.
lanowski J P and O'Donell M J (2004) Basolateral ion transport mechanisms during fluid secretion by Drosophila Malpighian tubulus: Na+ recycling, Na+,K+,2Cl- cotransport and Cl- conductance J Exp Biol 207: 2599-2609.
Wieczorek H, et al. (2009) Vacuolar-type proton pumps in insect epithelia, J Exp Biol. 212:1611-9.

## Claims

1. A method for assessing a pharmaco-kinetic profile of a chemical compound, such as a drug, in vertebrates, said method comprises the steps of:
• determining the concentration of a chemical compound in the hemolymph samples periodically taken from an insect in which a chemical compound has been administered intra intestinally, into the hemolymph or via oral administration; and
• plotting the PK profile of the chemical compound.

2. The method of claim 1, wherein the insect is selected from the group consisting of the orders Dictyoptera, Orthoptera, Cheleutoptera, Hymenoptera, Odonata, Lepidoptera and Diptera.

3. The method of claim 1 or 2, wherein the concentration of the chemical compound is determined by LC/MS.

4. The method of any one of the claims 1-3, wherein the chemical compound is administered to the insect and the fate of the compound is studied for 1 min to 72 hours.

5. The method of any one of the claims 1-4, wherein the insect is an adult insect, preferably an adult insect selected from the order Acridoidea.

## Patentansprüche

1. Verfahren zur Beurteilung eines pharmakokinetischen Profils einer chemischen Verbindung, wie beispielsweise eines Arzneimittels, in Vertebraten, wobei das Verfahren die folgenden Schritte umfasst:
- Bestimmung der Konzentration einer chemischen Verbindung in den Hämolympheproben, die von einem Insekt periodisch aufgenommen sind, welchem Insekt eine chemische Verbindung intra-intestinal, in die Hämolymphe oder durch orale Verabreichung verabreicht worden ist; und
- Plotten des Profils (PK) der chemischen Verbindung.

2. Verfahren nach Anspruch 1, wobei das Insekt aus der Gruppe bestehend aus den Ordnungen Dictyoptera, Orthoptera, Cheleutoptera, Hymenoptera, Odonata, Lepidoptera und Diptera ausgewählt ist.

3. Verfahren nach Anspruch 1 oder 2, wobei die Konzentration der chemischen Verbindung durch LC/MS bestimmt wird.

4. Verfahren nach einem der Ansprüche 1-3, wobei dem Insekt die chemische Verbindung verabreicht wird, und das Schicksal der Verbindung für 1 Minuten bis 72 Stunden untersucht wird.

5. Verfahren nach einem der Ansprüche 1-4, wobei das Insekt ein erwachsenes Insekt ist, vorzugsweise ein aus der Ordnung Acridoidea ausgewähltes erwachsenes Insekt ist.

## Revendications

1. Procédé pour évaluer un profil pharmacocinétique d'un composé chimique, tel qu'un médicament, chez les vertébrés, ledit procédé comprenant les étapes consistant à:
• déterminer la concentration d'un composé chimique dans les échantillons d'hémolymphe pris périodiquement à partir d'un insecte dans le-quel le composé chimique a été administré de manière intra intestinale, dans l'hémolymphe ou par administration orale; et
• tracer le profil pharmacocinétique du composé chimique.

2. Procédé selon la revendication 1, dans lequel l'insecte est choisi dans le groupe constitué par les ordres Dictyoptères, Orthoptères, Chéleutoptères, Hyménoptères, Odonates, Lépidoptères et Diptères.

3. Procédé selon la revendication 1 ou 2, dans lequel la concentration du composé chimique est déterminée par CL / SM.

4. Procédé selon l'une quelconque des revendications 1 à 3, dans lequel le composé chimique est administré à l'insecte et le destin du composé est étudié pour une période comprise entre 1 minute et 72 heures.

5. Procédé selon l'une quelconque des revendications 1 à 4, dans lequel l'insecte est un insecte adulte, préférablement un insecte adulte choisi dans l'ordre Acridoides.
